(19) Europäisches Patentamt
European Patent Office
Office européen des brevets

(11) Publication number: **0 178 929**
**B1**

(12) # EUROPEAN PATENT SPECIFICATION

(45) Date of publication of patent specification: **22.03.89**

(51) Int. Cl.⁴: **C 07 C 69/16**, C 07 C 67/42, C 07 C 45/54, C 07 C 45/46, C 07 C 27/02

(21) Application number: **85307489.6**

(22) Date of filing: **17.10.85**

(54) **Process for producing aromatic diols and their ester and ether derivatives.**

(30) Priority: **17.10.84 US 661552**

(43) Date of publication of application:
**23.04.86 Bulletin 86/17**

(45) Publication of the grant of the patent:
**22.03.89 Bulletin 89/12**

(84) Designated Contracting States:
**AT BE CH DE FR GB IT LI LU NL SE**

(56) References cited:
**EP-A-0 060 419**

**THE JOURNAL OF THE AMERICAN CHEMICAL SOCIETY, vol. 72, September-December 1950, Easton, PA, W. VON E. DOERING et al. "The peracetic acid clearage of unsymmetrical Ketones"**

**CHEMICAL ABSTRACTS, vol. 93, no. 1, July 7, 1985, Columbus, Ohio, USA, N. HIRAO et al., "Synthesis of dihydrobenzene by oxidation of hydroxy acetophenone", p. 711, abstract no. 7777n**

(73) Proprietor: **CELANESE CORPORATION**
**1211 Avenue of the Americas**
**New-York New York 10036 (US)**

(72) Inventor: **Gerberich, Harold R.**
**4814 Augusta Drive**
**Corpus Christi Texas (US)**

(74) Representative: **De Minvielle-Devaux, Ian Benedict Peter et al**
**CARPMAELS & RANSFORD 43, Bloomsbury Square**
**London WC1A 2RA (GB)**

(56) References cited:
**THE JOURNAL OF THE AMERICAN CHEMICAL SOCIETY, vol. 62, January-June 1940, Easton, PA, J.H. SIMONS et al. "Hydrogen fluoride as a condensing agent. X. Rearrangements"**

**THE JOURNAL OF THE AMERICAN CHEMICAL SOCIETY, vol. 61, July-December 1939, Easton, PA, J.H. SIMONS et al. "Hydrogen fluoride as a condensing agent. VII. The acylation of aromatic compounds"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 178 929 B1

**Description**

This invention relates to a process for the production of certain aromatic diol derivatives (of the class of carboxylate ester and monoalkyl ether derivatives) and aromatic diols themselves.

Aromatic diols such as hydroquinone are known commodities of commerce having use, for example, as photographic developers, polymerization inhibitors, dye intermediates and anti-oxidants.

Hydroquinone (HQ) has been commercially produced by the manganese oxide-catalyzed oxidation of aniline to quinone followed by the iron-catalyzed reduction of the quinone to hydroquinone. A more recently developed method of producing hydroquinone comprises the diisopropylation of benzene to form para-diisopropylbenzene followed by peroxidation of the latter compound and acid-catalyzed decomposition of the diperoxide to produce hydroquinone and acetone. While there are advantages in each of these processess, a process of making aromatic diols such as hydroquinone which involved a lower cost of construction of a commercial unit and/or a lower operating cost of production would be highly desirable.

The carboxylate esters and monoalkyl ethers of the aromatic diols are also known. In addition to some of the same uses mentioned for aromatic diols, these compounds have use as bactericides and antiseptics, pharmaceuticals e.g. as expectorants and depigmentors, and fragrances.

Aromatic diols and their dicarboxhylate esters, e.g. the diacetate ester of hydroquinone (DAHQ), have also found application as monomers in the preparation of certain polymers, e.g. those capable of forming an anisotropic melt phase and suitable for being formed into shaped articles such as molded articles, fibers and films as described, for example, in U.S. Patents Nos. 4,330,457; 4,351,918; and 4,355,132.

With regard to the process disclosed and claimed herein, the oxidation of ketones with peroxy compounds to form esters, i.e. the "Baeyer-Villiger" reaction, is known. Some references with disclosures of examples of this type of reaction and descriptions of their teachings are as follows:

Hirao et al, Koen Yoshishu- Koryo, Terupen oyobi Seiyu Kagaku ni kansuru Toronkai, 23rd 1979, 131—3 (Japan). Chem. Soc. Japan: Tokyo, Japan, as abstracted in C.A. *93*, 7777n, (1980) show the Fries rearrangement of phenyl acetate and friedel-Crafts acetylation of phenol to form para-hydroxy-acetophenone which is then oxidized with hydrogen peroxide using sulfuric acid as catalyst to para-hydroxyphenyl acetate; the latter is then hydrolyzed with sulfuric acid in acetic acid to yield hydroquinone.

Von E. Doering et al, Journal of the American Chemical Society *72*, 5515—5518 (1950) teach the Baeyer-Villiger oxidation of various aromatic ketones, including acetophenone and para-substituted benzophenones, to esters using peracetic acid as the oxidant and show that sulfuric acid has a "marked catalytic action" with regard to the yield and reaction time (page 5517, col. 1).

Ogata et al, Journal of Organic Chemistry *43* No. 12, 2417—2419 teach the Baeyer-Villiger oxidation of acetophenone and substituted acetophenones to the corresponding aryl acetates using permonophosphoric acid as oxidant and sulfuric acid as catalyst and show that the reaction rate is highly dependent on the acidity of the solution.

Starcher et al, Journal of the American Chemical Society, *80*, 4079—4082 (1958) teach the synthesis of lactones by means of the Baeyer-Villiger oxidation of cyclic ketones using peracetic acid as oxidant. In the paragraph bridging pages 4079 and 4080, the authors state: "The absence of inorganic impurities, notably water, hydrogen peroxide, mineral acids and salts, reduced polymerization to a minimum during the reaction step and avoided many of the by-products which plagued previous investigators."

Adam et al, Journal of Organic Chemistry, *44*, No. 26, 4969 (1979); describe the use of bis(trimethylsilyl) monoperoxysulfate as a Baeyer-Villiger oxidant in the oxidation of ketones to their corresponding esters. The authors state that the shortcomings of a previously-used oxidant for this purpose, Caro's or monoperoxysulfuric acid, are "(i) the use of aqueous conditions, (ii) the presence of strong acid and (iii) undesirable side reactions."

In addition to Hirao et al discussed previously, other references showing the formation of hydroxyaromatic ketones by the Fries rearrangement of aromatic esters or the Friedel-Crafts acylation of phenols are also in the literature. The following are some of these references with summaries of their teachings:

Lewis, U.S. Patent No. 2,833,825 shows the rearrangement of phenyl or other aromatic esters to acylphenols or other hydroxy aromatic ketones using anhydrous hydrogen fluoride as catalyst. The examples of this patent are limited to the rearrangement of esters of higher fatty acids with the yields ranging from 55 to 95%.

Simons et al, Journal of the American Chemical society, *62*, 485 and 486 (1940) show the use of hydrogen fluoride as a condensing agent for various rearrangements and at page 486 show the Fries rearrangement of phenyl acetate to obtain p-hydroxyacetophenone.

Dann and Mylius in a dissertation included as part of a series of Reports from the Institute for Applied Chemistry of the University of Erlangen, received for publication on January 7, 1954 and published in Annalen der Chemie 587 Bank, pages 1 to 15, show the rearrangement of phenyl acetate in hydrogen fluoride to 4-hydroxyacetophenone, with a maximum yield of 81% after 24 hours of reaction time, and report a yield of 92% stated to be obtained by K. Weichert as reported in Angewandte Chemie *56*, 338 (1943). However, Dann and Mylius suggest that the difference in yields may be at least partly due to the previous ignoring by Weichert of the accompanying 2-hydroxyacetophenone. Dann and Mylius also report

somewhat lower yields of hydroxy aromatic ketones from rearrangement in hydrogen fluoride of m-cresyl acetate, p-cresyl acetate, and guaiacol acetate.

Dann and Mylius also disclose the reaction of phenol and glacial acetic acid in the presence of hydrogen fluoride to produce 4-hydroxyacetophenone in a yield of 61.6%. This reaction may be conventionally characterized as a Friedel-Crafts actylation of phenol with acetic acid as the acetylating agent.

Simons et al, Journal of the American Chemical Society, *61,* 1795 and 1796 (1939) teach the acylation of aromatic compounds using hydrogen fluoride as a condensing agent and in Table 1 on page 1796 show the acetylation of phenol with acetic acid to produce p-hydroxyacetophenone in 40% yield.

Meussdoerffer et al, German Offenlegungsschrifft 26 16 986 published October 27, 1977 and assigned to Bayer AG, disclose the acylation of phenolic compounds such as phenol itself with an acyl halide such as acetyl chloride to form hydroxy aromatic ketones.

In accordance with this invention, a product comprising a major proportion of a carboxylate ester of an aromatic diol, e.g. the monoacetate of hydroquinone (MAHQ), or a carboxylate ester of a monoalkyl ether of an aromatic diol, e.g. 4-acetoxyanisole, is produced by subjecting an aromatic ketone, containing a hydroxy, alkoxy or acyloxy radical on the aromatic ring, e.g. 4-hydroxy-acetophenone (4-HAP), to a "Baeyer-Villiger" oxidation using as oxidant peracetic acid under conditions such that the reaction mass contains no more than 0.1 weight percent of sulfuric acid based on the weight of the initially added pure peracetic acid.

Preferably, the reaction mass also contains no more than 5 weight percent of water based on the weight of pure peracetic acid initially added.

It is essential to this invention that there be *not* used the commercial peracetic acid solutions which contain sulfuric acid in an amount greater than the maximum amount defined herein and which also contain more than the preferred maximum amount of water defined herein, unless these commercially available solutions are subjected to treatment so that their contents of sulfuric acid and water are appropriately reduced.

This invention also contemplates the hydrolysis of carboxyalte esters of the aromatic diols, e.g. MAHQ, to obtain unmodified aromatic diols, e.g. hydroquinone (HQ), or monoalkyl ethers of aromatic diols.

The Baeyer-Villiger oxidation contemplated by this invention proceeds as in equation (I):

$$YO—Ar^1—\overset{\overset{O}{\|}}{C}R+CH_3CO_2OH \rightarrow YO—Ar^1—O\overset{\overset{O}{\|}}{C}R+CH_3COOH \qquad (I)$$

where R is an alkyl radical, $Ar^1$ is a divalent aromatic radical, (both more specifically defined below), and Y is hydrogen, R, or

$$R\overset{\overset{O}{\|}}{C}—.$$

If the monoacetate ester of hydroquinone (MAHQ) is the desired product, the reaction proceeds as in equation II:

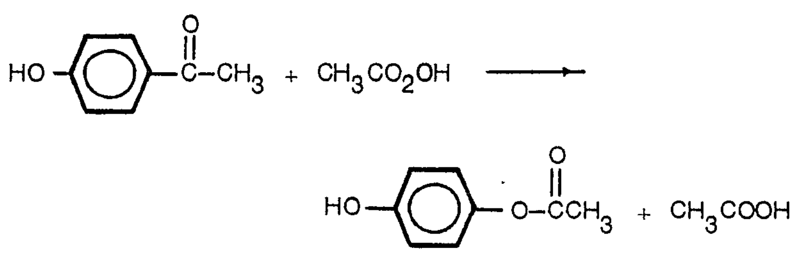

(II)

If a monocarboxylate ester of an aromatic diol is the product of the Baeyer-Villiger oxidation, it may be hydroylzed to the aromatic dio as shown in equation (III):

$$HOAr^1—O\overset{\overset{O}{\|}}{C}R+H_2O \xrightarrow{acid} HO—Ar^1—OH+HO\overset{\overset{O}{\|}}{C}R \qquad (II)$$

When it is desired to produce HQ from MAHQ, the hydrolysis reaction proceeds as in equation (IV):

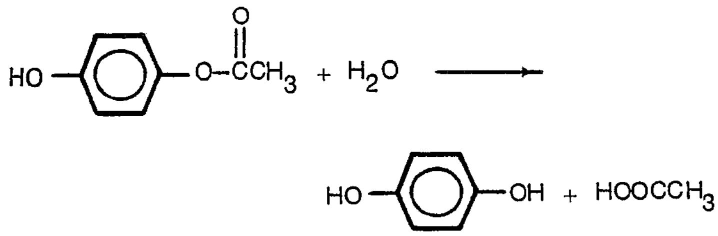

(IV)

In equations I and III, $Ar^1$ is a divalent aromatic radical. The specific nature of the radical is not critical but it is preferably a radical resulting from the removal of two ring hydrogen atoms from benzene, naphthalene, or biphenyl, either unsubstituted or with ring hydrogens subsituted with radicals such as methyl, tolyl; and appropriately masked amino, or sulfhydryl substituents. $Ar^1$ is preferably 1,4-phenylene, 2,1-naphthylene, 2,6-naphthylene, 5-phenyl-1,2-phenylene, 3-phenyl-1,4-phenylene or 3-methyl-1, 4-phenylene with the ketocarbon and corresponding groups occupying the first stated numbered position of $Ar^1$ when the positions are not equivalent. Most preferably $Ar^1$ is 1,4-phenylene.

The R groups in equations (I) and (III) are alkyl groups containing, for example, 1 to 18 carbon atoms, preferably 1 to 4 carbon atoms, and may be the same or different. More preferably, R is methyl, ethyl or propyl and most preferably methyl corresponding to the use of methyl ketones and others and acetate esters in the latter equations. The preferred products are the monoacetate ester of hydroquinone (MAHQ) formed by the Baeyer-Villiger oxidation of 4-hydroxyacetophenone (4-HAP) and hydroquionone (HQ) formed by the hydrolysis of the MAHQ.

The aromatic ketone used to form the carboxylate ester of an aromatic diol or the carboxylate ester of a monoalkyl ether of an aromatic diol may be prepared by any method known in the art. For example, a hydroxy aromatic ketone may be prepared by the Fries rearrangement of the corresponding aromatic ester as indicated by equation (V) where $Ar^1$ and R have the definitions given above and Ar is an aryl radical corresponding to the defination of $Ar^1$ given above except that the carbon bonded to hydroxy, alkoxy or acyloxy group are as shown in equation (I) is bonded to a hydrogen instead.

$$\mathrm{ArO}\overset{\mathrm{O}}{\overset{\|}{\mathrm{C}}}\mathrm{R} \xrightarrow{\text{catalyst}} \mathrm{HO{-}Ar^1{-}}\overset{\mathrm{O}}{\overset{\|}{\mathrm{C}}}\mathrm{R}, \qquad \text{(V)}$$

Alternatively, a phenolic compound and an acylating agent may be reacted in a Friedel-Crafts acylation to form the hydroxy aromatic ketone, in accordance with the following equation:

$$\mathrm{ArOH + R{-}}\overset{\mathrm{O}}{\overset{\|}{\mathrm{C}}}\mathrm{{-}X} \xrightarrow{\text{catalyst}} \mathrm{HO{-}Ar^1{-}}\overset{\mathrm{O}}{\overset{\|}{\mathrm{C}}}\mathrm{{-}R + HX} \qquad \text{(VI)}$$

where Ar, $Ar^1$ and R have the meanings given previously and X in equation (VI) is the residue minus the aryl group,

$$\mathrm{R{-}}\overset{\mathrm{O}}{\overset{\|}{\mathrm{C}}}\mathrm{{-}},$$

of compounds which are known acylating agents. X may be, for example, hydroxy, acyloxy, e.g., acetoxy; and halide, e.g., fluoride, chloride, and bromide. Examples of phenolic compounds which may be employed are phenol, 1-naphthol, 2-naphthol, 2-phenylphenol, 4-phenylphenol and o-cresol. Acylating agents which may be used are for example alkanoic acids, e.g., acetic and propionic acids, alkanoic acid anhydrides, e.g., acetic and propionic anhydrides, and acyl halides, e.g. acetyl and propionyl fluorides, chlorides, and bromides. It is to be noted that, although the reaction of a phenolic compound and an acylating agent is characterized herein as a "Friedel-Crafts acylation", no opinion as to the mechanism of reaction is implied by this characterization.

The catalyst for both of the foregoing reactions is preferably hydrogen fluoride but any other catalyst known in the art to be effective for the Fries and Friedel-Crafts reactions may be used, e.g. aluminum chloride, zinc chloride, or boron trifluoride.

In carrying out the reaction, the aromatic ester or phenolic compound and acylating agent, catalyst, and if desired (when an aromatic ester is the starting material) an additive for the reaction (such as acetic anhydride or acetic acid) may be charged into a corrosion-resistant reactor and the mixture maintained at a temperature, for example, of 20 to 150°C for a period, for example, of ½ to 4 hours, at a pressure, for example, of 25 to 500 psig (273.7 to 3548.8 kPa). If HF is used as the catalyst it may be charged as a liquid or a gas using well known handling methods.

In carrying out the reaction, an inert gas such as nitrogen may be used to keep the reaction space under the desired pressure and sufficient HF in contact with the reacting liquid. An excess of HF is generally used, for example, 7 to 75 moles per mole of aromatic ester of phenolic compound initially present in the reaction zone. If MAHQ is the desired product of the reaction, the starting material if a Fries rearrangement is employed will be phenyl acetate, while phenol and an acetylating agent such as acetic acid or acetic anhydride are the starting material if a Friedel-Crafts acylation is utilized. In both cases, the starting material is converted to 4-HAP which in turn converted by the process of this invention to MAHQ.

If a monoalkyl ether of an aromatic diol or the carboxylate ester of such ether is the desired product, the monoalkyl ether of the hydroxy aromatic ketone is the first prepared using any of the techniques known in the art, e.g., reaction of the hydroxy aromatic ketone, e.g. 4—HAP, with an alkyl iodide such as methyl iodide under basic conditions.

If a diester of an aromatic diol, e.g. the diacetate ester of hydroquinone (DAHQ), is desired as the end product, a hydroxy aromatic kethone such as 4-HAP may first be acylated, e.g. with acetic anhydride. (Such a method is described in our European Patent Application 85305222.3). The resulting acyloxy aromatic ketone, e.g., 4-acetoxyacetophenone (4-AAP), may then be subjected to a Baeyer-Villiger oxidation in accordance with this invention to produce a diester of an aromatic diol, e.g. DAHQ.

In producing esters of aromatic diols and esters of monoalkyl ethers of aromatic diols in accordance with equation (I), the aromatic ketone (e.g. the hydroxy aromatic ketone resulting from the previously described Fries re-arrangement of Friedel-Craft acylation) or a monoalkyl ether or monoester of such hydroxy aromatic ketone, is subjected to a Baeyer-Villiger oxidation by reacting the ketone with peracetic acid under such conditions that the reaction solution contains no more than 0.1 wt.%, preferably no more than 0.05 wt.% of sulfuric acid based on the weight of pure pereacetic acid initially added. Advantageously the reaction solution does not contain more than 5 wt.% (preferably not more than 2 wt.%) of water, based on the weight of pure peracetic acid. In general, the less the sulfuric acid and water present the better. The reaction solution may therefore be substantially free of sulfuric acid and/or water. A solvent for the hydroxy aromatic ketone may also be used in the initial reaction solution, e.g., an alkanoic acid such as glacial acetic acid or formic acid. Preferably, the peracetic acid is added in the form of a solution consisting essentially of 5 to 45 wt.% peracetic acid and the remainder a solvent such as acetic acid, methyl acetate, ethyl acetate, acetone or a mixture of any thereof.

The amount of peracetic acid supplied to the reaction is generally in the range for example of 0.5 to 2 moles per mole of aromatic ketone. The reaction may be carried out at a temperature, for example, of 20 to 140°C and a pressure, for example, of 25 mm of mercury to 2 atmospheres for a period, for example, of 0.5 to 3 hours.

An alternative method of producing diesters such as DAHQ is simply to subject the monester, e.g., MAHQ, resulting from the Baeyer-Villiger oxidation of hydroxy aromatic ketones e.g. 4-HAP, in accordance with this invention, to an acylation or esterification for example using techniques well-known in the art.

The following Examples further illustrate the invention.

Examples 1 to 5 illustrate the preparation of intermediates.

## Example 1

This Example illustrates the preparation of 4-hydroxyacetophenone by the Fries rearrangement of phenyl acetate using hydrogen fluorides as catalyst.

Into a 300 cc Hastelloy C autoclave was charged 40.8 g (0.3 mol) of phenyl acetate. The autoclave was sealed, immersed in a dry-ice/isopropanol bath and cooled internally to −45°C, and evacuated to ca. 100 Torr. 120 g (6.0 mol) of anhydrous hydrogen fluoride was added in a manner such that the internal temperature of the autoclave did not exceed 0°C. The internal pressure of the reactor was then adjusted to 0 psig (101.325 kPa) with nitrogen. The contents of the autoclave was stirred and heated to 75°C for 1 hour. The hydrogen fluoride was vented over a 45 minute period at ca. 45°C. The mixture was poured onto 25 g of ice and neutralized with 45% potassium hydroxide solution. The aqueous mixture was extracted with ethyl acetate. The organic fraction was then dried over anhydrous magnesium sulfate and filtered, and the solvent was removed on a rotary evaporator to yield 44.0 g of a dark green solid corresponding to 99.9% conversion of phenyl acetate and 94.3% selectivity to 4-hydroxyacetophenone.

## Example 2

This Example illustrates the preparation of 4-hydroxyacetophenone by the Fries rearrangement of phenyl acetate using hydrogen fluoride as catalyst with acetic anhydride as additive.

Into a 300 cc Hastelloy C autoclave was charged 50.6 g (0.5 mol) of acetic anhydride. The autoclave was cooled to −50°C and evacuated to 5 Torr. whereupon 120 g (6.0 mole) of anhydrous hydrogen fluoride was transferered from a cylinder to the autoclave. After the transfer of hydrogen fluoride has been completed, the internal temperature and the internal pressure of the autoclave were adjusted to −50°C and 0 psig (101.325 kPa) using nitrogen, respectively. Into the stirred autoclave were added 81.6 g (0.6 mol) of phenyl acetate at such a rate that the temperature of the mixture did not exceed −23°C. Upon completion of phenyl acetate addition, the contents were warmed to 50°C and stirred for 3 hours during which time a pressure of ca. 40 psig (377.125 kPa) was generated. At the end of the run, the hydrogen fluoride was vented through a caustic scrubber and the contents of the autoclave were poured onto ca. 30 g of ice. The pH of the mixture

was adjusted to 6.5 using 45% potassium hydroxide and the mixture was then extracted with 75 ml of ethyl acetate (3×). The organic solution was dried over anhydrous magnesium sulfate and filtered, and the solvent was removed using a rotary evaporator.

The reaction proceeded with 98.1% conversion of phenyl acetate and with the following selectivities: phenol, 1%; 4-hydroxyacetophenone (4-HAP), 82.3%; 2-hydroxyacetophenone (2-HAP), 4.3%; 3-hydroxyacetophenone (3-HAP), 0.1%; 4-acetoxyacetophenone (4-AAP), 3.8%; and 4-(4'-hydroxyphenyl)-acetophenone (HPAP), 0.4%.

Example 3

This Example illustrates the formation of 4-hydroxyacetophenone by the Fries rearrangement of phenyl acetate using hydrogen fluoride as catalyst and acetic acid as additive.

The procedure for Example 2 was repeated except that 18 grams (0.3 mole) of acetic acid rather than acetic anhydride were charged into the reactor before it was cooled and charged with the hydrogen fluoride. A conversion of 99.0% of phenyl acetate was obtained with the following selectivities: phenol, 3.3% 4-HAP, 80.8%; 3-HAP, 0; 2-HAP, 5.8%; 4-AAP, 0.3%; and HPAP, 0.3%.

Example 4

This Example illustrates the preparation of 4-hydroxyacetophenone (4-HAP) by the Friedel-Crafts acetalyation of phenol with acetic acid as the acetylating agent.

Phenol (9.4 g, 0.1 moles) and acetic acid (12.0 g, 0.2 moles) were charged into a 300 ml Hastelloy C autoclave at room temperature. The reactor was evacuated and cooled to −20°C. HF (100 g, 5 moles) was then transferred into the reactor. The reactor was heated to 80°C and maintained for 1 hour at reaction temperature. At the end of the reaction the reactor was cooled to 20°C and the excess HF was vented to a KOH scrubber. Ethyl acetate was added to the contents of the reactor. The mixture was then neutralized with 45% aqueous KOH. The resulting organic phase was separated, dried over $MgSO_4$ and evaporated to afford a yellow solid which contained 13.1 g (0.096 moles) of 4-HAP.

Example 5

This Example illustrates the preparation of 4-hydroxyacetophenone by the Friedel-Crafts acetylation of phenol with acetic anhydride as the acetylating agent.

Into a 300 cc Hastelloy C autoclave cooled to −20 to °C and evacuated to 50 Torr were charged 150 g (7.5 mole) of anhydrous hydrogen fluoride. The content of the autoclave was warmed to 50°C resulting in an internal pressure of 25 psig (273.7 kPa). A solution of 23.5 g (0.25 mole) of phenol and 25.5 g of acetic anhydride was added to the autoclave over a 3 minute period causing the pressure to drop to 14 psig (197.855). The solution was stirred for 1 hour at 50°C whereupon the hydrogen fluoride was vented at the reaction temperature. The contents of the autoclave were poured onto ice and the aqueous phase was adjusted to pH 6.0 with a 45% solution of potassium hydroxide. The aqueous phase was extracted with 75 ml of ethyl acetate (3×); the organic fractions were combined, dried over anhydrous magnesium sulfate, and filtered. The reaction proceeded with 99% conversion based on phenol and 95% selectivity to 4-hydroxyacetophenone.

The procedure of Examples 1 to 5 can also be used to produce, with high conversions and selectivities, 1-hydroxy-2-acetonaphthone from 1-naphthyl acetate; 6-hydroxy-2-acetonaphthone from 2-naphthyl acetate; 2-hydroxy-5-phenylacetophenone from 4-phenylphenyl acetate; 4-hydroxy-3-phenylacetophenone from 2-phenylphenyl acetate; and 4-hydroxy-3-methylacetophenone from 0-cresyl acetate.

Examples 6 to 12 illustrate the conversion of 4-hydroxy-acetophenone (4-HAP) to the monoacetate ester of hydroquinone by the Baeyer-Villiger oxidation, using peracetic acid as the oxidising agent under conditions defined by this invention.

Example 6

To 650 grams of commercially available peracetic acid were slowly added 650 grams of acetic anhydride. This commercial acid contained ca. 40 wt.% peracetic acid, ca. 13 wt.% water, ca. 5 wt% hydrogen peroxide and ca. 1 wt.% sulfuric acid, and the purpose of the acetic anhydride treatment was to convert the water and hydrogen peroxide present to acetic acid and peracetic acid, respectively. The solution was cooled and stirred during the addition to maintain the temperature at below 30°C. This solution was then distilled under vacuum (20—30 mmHgA, ca. 40°C) through a one-inch Vigreux column. The initial 20 cc of distillate were discarded. Then, ca. 1000 cc of material was distilled without reflux. This material referred to below as "purified peracetic acid solution" contained ca. 27 wt.% peracetic acid and ca. 73 wt.% acetic acid and was used for the oxidations in this and the following Examples.

In a solution of 60 grams of glacial acetic acid and 10 grams of acetic anhydride were dissolved 30 grams of 4-HAP. This solution was placed into a 250 cc three-neck flask. While heating the solution to 60°C, the flask was partially evacuated to a pressure of 60 mmHgA. This pressure was maintained throughout the experiment. To the solution were added 77.5 grams of the purified peracetic acid solution containing 20.46 g of pure peracetic acid, fed dropwise over a period of 40 minutes, and the solution was heated for an additional 80 minutes. The initial mole ratio of peracetic acid to 4-HAP was 1.22. The solution was then

cooled and analyzed by gas chromatography and other well-established procedures.

The product solution weighed 171.3 grams and contained 18.0 wt.% monoacetate ester of hydroquinone (MAHQ), 0.47 wt.% diacetate ester of hydroquinone (DAHQ), 0.03 wt.% hydroquinone (HQ), 0.05 wt.% benzoquinone (BQ), 0.17 wt.% 4-acetoxyacetophenone (4-AAP; and 0.41 wt.% of 4-hydroxyacetophenone (4-HAP). Peracetic acid was not detected. This corresponds to 97% conversion of 4-HAP and 97% efficiency to hydroquinone and its esters.

Example 7

The procedure of Example 6 was followed except that the temperature employed was 80°C, the solvent consisted of 70g acetic acid (and no acetic anhydride) to which 4.4 mg of ethylene diamine tetraacetic acid (EDTA) were added and 51.7 grams of purified peracetic acid solution containing 13.65 of pure peracetic acid were fed over the 40 min period. The initial mole ratio of peracetic acid to 4-HAP was 0.81.

The product solution weighed 154.0 grams and contained 13.7 wt.% MAHQ, 0.33 wt.% DAHQ, 0.78 wt.% HQ, 0.03 wt.% BQ, and 4.8 wt.% of 4-HAP, corresponding to 74.93% conversion of 4-HAP and 92.17% efficiency to HQ and its esters.

Example 8

The procedure of Example 6 was followed except that the solvent consisted of 70 grams of acetic acid and no acetic anhydride, to which 4.4 mg of EDTA were added, and the purified peracetic acid solution was added over a 10 minute period and the reaction solution was heated for an additional 40 minutes period. The initial mole ratio of peracetic acid to 4-HAP was 1.22.

The product solution weighed 177.3 grams and contained 16.8 wt.% MAHQ, 0.26 wt.% of DAHQ, and 0.13 wt.% HQ, 0.13 wt.% BQ and 0.02 wt.% 4-HAP, corresponding to 99.88% conversion of 4-HAP and 90.97% efficiency to HQ and its esters.

Example 9

The procedure of Example 7 was followed except that 51.7 grams of purified peracetic acid solution containing 14.37 grams of peracetic acid were fed over a 20 minute period, the solution was heated for an additional 40 minutes and the solvent consisted of 60 grams of acetic acid and 10 grams of acetic anhydride. The initial mole ratio of peracetic acid to 4-HAP was 0.86.

The product solution weighed 153.0 grams and contained 14.10 wt.% MAHQ, 1.10 wt.% of DAHQ, 0.77 wt.% HQ, 0.08 wt.% BQ and 4.60 wt.% 4-HAP corresponding to 75.41% conversion of 4-HAP and 97.97% efficiency to HQ and its esters.

Example 10

The procedures of Example 6 was followed except that 62.1 grams of purified peracetic acid solution containing 17.26 grams of peracetic acid were fed over a 30 minute period, the solution was heated for an additional 30 minutes, and the solvent consisted of 70 grams of acetic acid to which was added 0.1 gram of sodium acetate. The initial mole ratio of peracetic acid to 4-HAP was 1.03.

The product solution weighed 161.7 grams and contained 21.40 wt.% MAHQ, 0.00 wt.% DAHQ, 0.02 wt.% HQ, 0.00 wt.% BQ and 0.55 wt.% 4-HAP corresponding to a conversion of 4-HAP and 97.04% and an efficiency to HQ and its esters of 100%.

Example 11

The procedures of Example 6 was followed except that 107.4 grams of purified peracetic acid solution containing 29.11 grams of peracetic acid were fed over a 30 minute period, the solution was heated for an additional 30 minutes and the 4-HAP solution consisted of 50.8 grams of acetic acid and 50.8 grams of 4-HAP. The initial mole ratio of peracetic acid to 4-HAP was 1.04.

The product solution weighed 206.9 grams and contained 21.9 wt.% MAHQ, 0.09 wt.% DAHQ, 0.01 wt.% HQ and 1.70 wt.% 4-HAP corresponding to a normalized conversion of 4-HAP based on 88.85% accountability of products of 91.69% and a normalized efficiency to HQ and its esters of 99.9%.

Example 12

The procedures of Example 6 was followed except that 90.8 grams of purified peracetic acid solution containing 24.61 grams of peracetic acid were fed over a 30 minute period, the solution was heated for an additional 30 minutes and the 4-HAP solution consisted of 69.1 grams of 90% formic acid and 40.3 grams of 4-HAP. The initial mole ratio of peracetic acid to 4-HAP was 1.09

The product solution weighed 198.5 grams and contained 17.6 wt.% MAHQ, 0.17 wt.% DAHQ, 0.85 wt.% HQ, 0.04 wt.% BQ and 1.10 wt.% 4-HAP corresponding to a normalized conversion of 4-HAP based on 88.99% accountability of products of 93.91% and a normalized efficiency to HQ and its esters of 99.70%.

In comparative Examples A, B and C, 0.5 ml of concentrated sulfuric acid was added to the 4-HAP solution to determine the effect of the presence of a small amount of sulfuric acid such as that present in commercial peracetic acid solutions or used to catalyze Baeyer-Villager oxidations.

Comparative Example A

The procedures of Example 6 was followed except that the reaction temperature was 80°C, the 4-HAP solution contained 0.5 ml of concentrated sulfuric acid and 4.40 mg of EDTA, 77.5 grams of purified peracetic acid solution containing 21.54 grams of peracetic acid were fed over a 20 minute period and the solution was heated for an additional 100 minutes. The initial mole ratio of peracetic acid to 4-HAP was 1.28.

The product solution weighed 178.40 grams and contained 1.70 wt.% MAHQ, 0.21 wt.% DAHQ, 3.30 wt.% HQ, 0.01 wt.% BQ and 4.10 wt.% 4-HAP corresponding to a conversion of 4-HAP of 71.67% and an efficiency to HQ and its esters of 47.70%.

Comparative Example B

The procedure of Example 7 was followed except that the temperature of reaction was 60°C, the 4-HAP solution contained 0.5 ml of concentrated sulfuric acid, the purified peracetic acid solution was fed over a 20 minute period and no EDTA was employed.

The product solution weighed 148.60 grams and contained 2.00 wt.% MAHQ, 2.30 wt.% DAHQ, 7.00 wt.% HQ, 0.00 wt.% BQ and 6.80 wt.% 4-HAP corresponding to a conversion of 4-HAP of 64.27% and an efficiency to HQ and its esters of 92.91%.

Comparative Example C

The procedure of Example 9 was followed except that the reaction temperature of reaction was 60°C, then 4-HAP solution contained 0.5 ml of concentrated sulfuric acid and 4.4 mg EDTA, the paracetic acid was fed over a 40 minute period, and the solution was heated for an additional 20 minute period.

The product solution weighed 153.20 grams and contained 3.10 wt.% MAHQ, 0.31 wt.% DAHQ, 0.03 wt.% BQ and 5.00 wt.% 4-HAP corresponding to a conversion of 4-HAP of 64.32% and an efficiency to HQ and its esters of 67.92%.

A comparison of the results obtained in Examples 6, 7 and 9, wherein no sulfuric acid was present in the reaction with those obtained in comparative examples A, B and C wherein a small amount of sulfuric acid was present comparable to that present in commercial peracetic acid solutions or added as a catalyst in Baeyer-Villiger oxidations, indicates that the absence of any appreciable amount of sulfuric acid has an unexpectedly positive effect on the oxidation of hydroxy aromatic ketones such as 4-HAP, to aromatic diols and their esters, such as MAHQ, DAHQ and HQ, both with regard to the conversion of the ketone and efficiency to the diols and their esters.

The following comparative Example D illustrates the effect of an appreciable amount of water in the oxidation reaction solutions.

Comparative Example D

The procedure of Example 10 was followed except that 10 grams of water were added to the 4-HAR solution and no sodium acetate was added.

The product solution weighed 167.00 grams and contained 21.10 wt.% MAHQ, 0.00 wt.% DAHQ, 0.13 wt.% HQ, 0.00 wt.% BQ and 1.30 wt.% HAP corresponding to a conversion of 4-HAP of 92.76% and an efficiency to HQ and its esters of 100%.

Comparison of the results of comparative Example D with those of Example 10 shows that he presence of an appreciable amount of water in the reaction has the effect of reducing somewhat the conversion of the hydroxy aromatic ketone to the diol ester.

Comparative Examples E and F illustrate the effect of using hydrogen peroxide rather than peracetic acid as the oxidant.

Comparative Example E

The procedure of Example 11 was followed except that 26.6 grams of a hydrogen peroxide solution described in comparative Example E were used as the oxidant in place of the peracetic acid solution and the 4-HAP solution consisted of 53.2 grams of 90% formic acid and 30.0 grams of 4-HAP. The initial mole ratio of hydrogen peroxide to 4-HAP was 1.09.

The product solution weighed 105.2 grams and contained 6.00 wt.% MAHQ, 0.0 wt.% DAHQ, 8.10 wt.% HQ, 0.0 wt.% BQ, and 5.00 wt.% 4-HAP corresponding to a normalized conversion of 4-HAP fo 75.47% and a normalized efficiency to HQ and its esters of 100.00% based on 71.48% accountablity of products.

Comparison of the results of Comparative Examples E and F with those of Examples 11 and 12 indicates that the use of peracetic acid in the absence of sulfuric acid as oxidant yields higher conversions of 4-HAP than hydrogen peroxide with equivalent efficiencies of HQ and its esters.

Example 13

This Example illustrates the hydrolysis of MAHQ and DAHQ or HQ.

Product from several experiments as described in Example 6 were combined and most of the acetic acid removed by evaporation under vacuum at 100°C. Analysis of the non-volatile portion showed that it contained 81.5 wt.% MAHQ, 2.0 wt.% HQ, 2.2 wt.% DAHQ, 1.5 wt.% 4-HAP, 0.01 wt.% BQ, and 1.2 wt.% acetic acid. 40.0 grams of this non-volatile material were added to a solution of 0.4 grams of 37 wt.%

hydrochloric acid in 40.0 grams of water and heated for two hours at 85°C. Upon cooling in ice-water a crystalline solid precipitated. The solid was filtered, washed with water, and dried in a vacuum desiccator. The dried solid weighed 14.02 grams and contained 97.7 wt.% HQ, 0.05 wt.% MAHQ, 0.0 wt.% DAHQ, 0.58 wt.% BQ, 2.4 wt.% acetic acid, 0.55 wt.% water, and 0.0 wt.% 4-HAP. The filtrate plus wash weighed 99.42 grams and contained 11.3 wt.% HQ, 0.10 wt.% MAHQ, 0.02 wt.% DAHQ, 17.0 wt.% acetic acid, 70.6 wt.% water, 0.01 wt.% BQ, and 0.23 wt.% 4-HAP. These results show that of the total MAHQ, DAHQ, and HQ fed (0.2263 mole) 99.4% (0.2249 mole) was recovered as HQ in the solid and filtrate.

Example 14

This example illustrates the Baeyer-Villiger oxidation of 4-acetoxyacetophenone (4-AAP) to primarily DAHQ.

The procedure of Example 6 was followed except that 15.0 grams of 4-AAP were dissolved in 50 grams of acetic acid (no acetic anhydride), the temperature employed was 80°C, 26.1 grams of purified peracetic acid solution were added over a 30 minute period, and the reaction solution was heated for an additional 30 minute period. The initial mole ratio of peracetic acid to 4-AAP was 1.10.

The product solution weighed 91.0 grams and contained 2.84 wt.% DAHQ, 0.01 wt.% MAHQ, 0.03 wt.% HQ, 0.01 wt.% BQ, 14.31 wt.% 4-AAP, and 5.42 wt.% peracetic acid, corresponding to a normalized conversion of 4-AAP of 16.35% and a normalized efficiency to HQ and its esters of 98.90% based on 103.17% accountability of products.

The product solution was subjected to further oxidation following the procedure of Example 6 except that 87.3 grams of product solution were added to the reaction flask, the temperature employed was 95°C, 25 grams of purified peracetic acid solution were added over a 30 minute period, and the reaction solution was heated for an additional 90 minute period. The initial mole ratio of peracetic acid to 4-AAP was 2.19.

The final product solution weighed 110.3 grams and contained 7.80 wt.% DAHQ, 0.05 wt.% MAHQ, 0.01 wt.% HQ, 0.01 wt.% BQ, 6.90 wt.% 4-AAP, and 0.60 wt.% peracetic acid, corresponding to a normalized conversion of 4-AAP of 53.30% and a normalized efficiency to HQ and its esters of 99.77% based on 104.03% accountability of products.

Example 15

This Example illustrates the methylation of 4-hydroxyacetophenone (4-HAP) to form p-methoxyacetophenone (PMAP).

50 g (0.37 mol) of 4-hydroxyacetophenone, 51.8 g (0.37 mol) of methyl iodide, and 51 g (0.37 mol) of potassium carbonate were dissolved in 55 ml of dry acetone and gently refluxed for 24 hours. After cooling the reaction mixture 200 ml of water were added to dissolve the precipitated potassium iodide. The resulting mixture was extracted three times with 200 ml of ether. The combined organic material was separated, washed with 10% sodium hydroxide dried over anhydrous potassium carbonate, filtered and evaporated. Distillation of the residue (110°C @ 2 mmHgA) afforded 50 g (ca. 92% yield) of a white crystalline solid which was p-methoxyacetophenone (PMAP).

Example 16

This Example illustrated the Baeyer-Villiger oxidation of p-methoxyacetophenone (PMAP) to p-methoxyphenyl acetate (PMPA).

The procedure of Example 6 was followed except that 24.2 grams of PMAP and 0.005 gram of EDTA were dissolved in 76.8 grams of acetic acid (no acetic anhydride), 45.7 grams of purified peracetic acid solution were added over a 20 minute period, and the reaction solution was heated for an additional 60 minute period. The initial mole ratio of peracetic acid to PMAP was 1.11.

The product solution weighed 144.2 grams and contained 13.70 wt:% PMPA, 0.01 wt.% p-methoxyphenol, i.e. the monmethyl ether of hydroquinone (MEHQ), 4.30 wt.% PMAP, and 0.90 wt.% peracetic acid, corresponding to 74.40% conversion of PMAP and 99.9% efficiency to PMPA.

The PMPA may be hydrolyzed to MEHQ using the procedure of Example 13.

The procedures of Examples 6 to 16 can also be used to prepare, for example, 3-phenylcatechol from 2-hydroxy-5-phenyl-acetophenone; phenylhydroquinone from 4-hydroxy-3-phenyl-acetophenone; methylhydroquinone from 4-hydroxy-3-methyl-acetophenone; catechol from 2-hydroxyacetophenone (2-HAP); guaiacol from 2-methoxyacetophenone; 2,6-diacetoxynaphthalene and 2,6-dihydroxynaphthalene (2,6-DHN) from 6-acetoxy-2-acetonaphthone; and 4-acetoxy-4'-hydroxybiphenyl and 4,4'-biphenol from 4(4'-hydroxyphenyl) acetophenone.

It will of course be understood that the present invention has been described above by way of example, and that modifications of detail can be made within the scope of the invention.

**Claims**

1. A process in which there is produced a carboxylate ester of an aromatic diol or a carboxylate ester of a monoalkyl ether of an aromatic diol, which comprises subjecting an aromatic ketone (containing a hydroxy, alkoxy or acyloxy radical on the aromatic ring) to a Baeyer-Villiger oxidation using peracetic acid as oxidant such that the reaction mass contains no more than 0.1 wt.% of sulfuric acid based on the weight

of peracetic acid initially added.

2. The process of claim 1 in which the reaction mass contains no more than 5 wt.% of water based on the weight of peracetic acid initially added.

3. The process of claim 1 or 2 in which the said aromatic ketone is 4-hydroxyacetophenone and the said carboxylate ester is the monoacetate ester of hydroquinone.

4. The process of claim 1 or 2 in which the said aromatic ketone containing a hydroxy radical is prepared by contacting an ester of a phenolic compound and carboxylic acid, with a Fries rearrangement catalyst.

5. The process of both claims 3 and 4 in which the said ester of a phenolic compound is phenyl acetate, and the said Fries rearrangement catalyst is hydrogen fluoride.

6. The process of claim 1 or 2 in which the said aromatic ketone containing a hydroxy radical is prepared by contacting a phenolic compound and an acylating agent with a Friedel-Crafts catalyst.

7. The process of both claims 3 and 6 in which the said phenolic compound is phenol, said acylating agent is acetic acid or acetic anhydride, and the said Freidel-Crafts catalyst is hydrogen fluoride.

8. The process of any of claims 1—7 in which the said carboxylate ester is hydrolyzed to an aromatic diol or a monoalkyl ether of an aromatic diol.

9. The process of both claims 3 and 8 in which the said monoacetate ester is hydrolyzed to hydroquinone.

**Patentansprüche**

1. Verfahren zur Herstellung eines Carboxylatesters eines aromatischen Diols oder eines Carboxylatesters eines Monoalkylethers eines aromatischen Diols, dadurch gekennzeichnet, daß man ein aromatisches Keton (enthaltend einen Hydroxy-, Alkoxy- oder Acyloxyrest am aromatischen Ring) einer Baeyer-Villiger-Oxidation unter Verwendung von Peressigsäure als Oxidationsmittel, derart, daß die Reaktionsmasse nicht mehr als 0,1% Schwefelsäure, bezogen auf auf das Gewicht der anfangs zugesetzten Peressigsäure, enthält, unterwirft.

2. Verfahren nach Anspruch 1, dadurch gekennzeichnet, daß die Reaktionsmasse nicht mehr als 5% Wasser, bezogen auf die anfangs zugesetzte Peressigsäure, enthält.

3. Verfahren nach Anspruch 1 oder 2, dadurch gekennzeichnet, daß das aromatische Keton 4-Hydroxyacetophenon und der Carboxylatester der Monoacetatester von Hydrochinon ist.

4. Verfahren nach Anspruch 1 oder 2, dadurch gekennzeichnet, daß das einen Hydroxyrest enthaltende aromatische Keton hergestellt wird, indem man einen Ester einer phenolischen Verbindung und eine Carbonsäure mit einem Fries-Umlagerungskatalysator in Berührung bringt.

5. Verfahren nach den Ansprüchen 3 und 4, dadurch gekennzeichnet, daß der Ester einer phenolischen Verbindung Phenylacetat und der Fried-Umlagerungskatalysator Fluorwasser ist.

6. Verfahren nach Anspruch 1 oder 2, dadurch gekennzeichnet, daß das einen Hydroxyrest enthaltende aromatische Keton hergestellt wird, indem man eine phenolische Verbindung und ein Acylierungsmittel mit einem Freidel-Crafts-Katalysator in Berührung bringt.

7. Verfahren nach den Ansprüchen 3 und 6, dadurch gekennzeichnet, daß die phenolische Verbindung Phenol, das Acylierungsmittel Essigsäure oder Essigsäureanhydrid und der Freidel-Crafts-Katalysator Fluorwasserstoff ist.

8. Verfahren nach einem der Ansprüche 1 bis 7, dadurch gekennzeichnet, daß der Carboxylatester zu einem aromatischen Diol oder einem Monoalkylether eines aromatischen Diols hydrolysiert wird.

9. Verfahren nach den Ansprüchen 3 und 8, dadurch gekennzeichnet, daß der Monoacetatester zu Hydrochinon hydrolysiert wird.

**Revendications**

1. Procédé où l'on produit un ester de carboxylate d'un diol aromatique ou un ester de carboxylate d'un monoalkyl éther d'un diol aromatique, qui consiste à soumettre une cétone aromatique (contenant un radical hydroxy, alcoxy ou acyloxy sur le noyau aromatique) à une oxydation de Baeyer-Villiger en utilsant l'acide peracétique comme oxydant, de manière que la masse réactionnelle ne contienne pas plus de 0,1% en poids d'acide sulfurique en se basant sur le poids de l'acide peracétique initialement ajouté.

2. Procédé selon la revendication 1 où la masse réactionnelle ne contient pas plus de 5% en poids d'eau en se basant sur le poids de l'acide peracétique initialement ajouté.

3. Procédé selon la revendication 1 ou 2 dans lequel ladite cétone aromatique est la 4-hydroxyacétophénone et ledit ester de carboxylate est l'ester de monoacétate d'hydroquinone.

4. Procédé selon la revendication 1 ou 2 où ladite cétone aromatique contenant un radical hydroxy est préparée par contact d'un ester d'un composé phénolique et d'acide carboxylique, avec un catalyseur de réarrangement de Fries.

5. Procédé selon les revendications 3 et 4 où ledit ester d'un composé phénolique est l'acétate de phényle et ledit catalyseur de réarrangement de Fries est le fluorure d'hydrogène.

6. Procédé selon la revendication 1 ou 2 où ladite cétone aromatique contenant un radical hydroxy est

préparée par mise en contact d'un composé phénolique et d'un agent acylant avec un catalyseur de Friedel-Crafts.

7. Procédé selon les revendications 3 et 6 où ledit composé phénolique est le phénol, ledit agent acylant est l'acide acétique ou l'anhydride acétique et ledit catalyseur de Friedel-Crafts est le fluorure d'hydrogène.

8. Procédé selon l'une des revendications 1—7 où ledit aster de carboxylate est hydrolysé en un diol aromatique ou un monoalkyl éther d'un diol aromatique.

9. Procédé selon les revendications 3 et 8 où ledit ester de monoacétate est hydrolysé en hydroquinone.